# EUROPEAN PATENT SPECIFICATION

(11) **EP 2 014 220 B1**
(45) Date of publication and mention of the grant of the patent: **19.12.2012**
(21) Application number: 07112034.9
(22) Date of filing: 09.07.2007
(51) Int. Cl.: A61B 1/227, A61B 5/107

(54) **Apparatus for obtaining geometrical data relating to a cavity**
Vorrichtung für den Erhalt geometrischer Daten in Zusammenhang mit einem Hohlraum
Appareil pour obtenir les données géométriques liées à une cavité

(43) Date of publication of application: 14.01.2009
(73) Proprietor: Oticon A/S, 2765 Smørum (DK); Widex A/S, 3500 Værløse (DK)
(72) Inventor: Søgaard, Jan, DK2765, Smørum (DK); Foged, Peter, DK-2765, Smørum (DK)
(74) Representative: Nielsen, Hans Jörgen Vind

(56) References cited:
- WO-A-97/14932
- WO-A-02/091920
- WO-A-2007/045696
- US-A- 5 895 927
- US-A1- 2007 081 168

## Description

### TECHNICAL FIELD

The present invention is related to a method and an apparatus for obtaining geometrical data relating to the internal surface of a cavity, such as the ear and ear canal of the human body.

### BACKGROUND

An interesting application of such a method and apparatus includes the generation of a data mapping of the internal surface of the ear and ear canal, so that 3-dimensional data or a digital model of the internal surface of the ear and ear canal can be obtained. Such a 3-dimension model can be used to produce a shell, which has the exact shape of the canal and the shell may form the basis for an In-The-Ear (ITE) or Completely-In-The-Canal (CIC) hearing aid. Also ear moulds or shells for other purposes such as a hearing protection or for headsets may be produced from the data model. The shell can be produced on the basis of the data model in different ways, such as by recent developed rapid prototyping methods, stereolithography (SLA) or by well known machining, e.g. in a Computer Numerically Controlled (CNC) machining centre.

The advantage of having a data model of the ear canal, e.g. compared to traditional manufacturing processes based on ear impressions taken by introducing a semi-fluent, curable material into the ear canal, is easy and fast data exchange and a shorter delivery time of the final shell. Further so the data model may be transmitted either as it is obtained or right thereafter for evaluation at a production facility. Thereby a data model of the hearing aid may be generated, which may be realized based on the dimensions and shape of the canal. The data model of the hearing aid can be transmitter back to the end user for visual evaluation.

International patent application WO 02/091920 discloses a method and apparatus for obtaining geometrical data relating to the internal surface of the ear and ear canal of the human body in order to be able to generate a model of the internal surface of the ear and ear canal. To this end this prior art document discloses a probe having a longitudinal axis, where light is emitted from a mirror at the tip of the probe in a right angle away from the longitudinal axis of the probe and towards the surrounding canal wall. The apparatus comprises a detector for detecting reflected sight from the canal wall, and analysing means for determining the distance from the probe to the internal surface of the canal at points of the circumference.

However, it remains a problem to provided accurate measurements in a region close to the end wall of a cavity, e.g the tympanic membrane when the cavity is an ear canal. In particular, the probe has to be inserted very close to the end wall in order to be able to measure the circumferential surface of the ear canal close to the tympanic membrane, thereby involving the risk of touching the tympanic membrane which may be unpleasant or even harmful for the person under examination.

Even though WO 02/091920 further discloses that the mirror may be semitransparent, such that the light in a first wavelength range is directed parallel to the longitudinal axis so as to provide a natural image of objects in front of the probe, this additional light may reduce the efficiency of the measurement of the circumferential surface, since some of the light intensity is used for the optical control rather than the desired measurement.

WO 97/14932, WO 2007/045696 A2 and US 2007/0081168 A1 each disclose an apparatus for obtaining geometrical data relating to an internal surface of a cavity, wherein the apparatus comprises a probe, detection means for detecting reflected radiation and means for determining from that reflected radiation a distance between the probe and a surface of the cavity.

### SUMMARY

The invention is as disclosed in claim 1.

According to one aspect, disclosed herein is an apparatus for obtaining geometrical data relating to an internal surface of a cavity. The apparatus comprises a probe having an end portion insertable into the cavity in a direction of insertion, radiation directing means for directing electromagnetic radiation from the end portion to at least one location on the internal surface to cause the radiation to be reflected from said location, detection means for detecting the reflected radiation from the at least one location, and means for determining from the detected radiation a distance between the probe and the internal surface in a direction having at least a transverse component relative to the direction of insertion. The radiation directing means is adapted to direct the radiation at an acute angle relative to the direction of insertion and, the radiation directing means is adapted to direct the radiation from an exit position radially displaced from the longitudinal axis. The at least one location and the exit position are located on opposite sides of a plane through the longitudinal axis, the plane having a normal along the direction of the radial displacement of the exit position.

For example, the radiation directing means may be adapted to direct a plurality of beams of radiation from the distal end portion to respective locations on the internal surface the plurality of beams of radiation intersect in a position displaced from the distal end portion in the direction of insertion so as to define a cone around the direction of insertion. In one embodiment, the beams define a cone or a double cone having an apex displaced from the distal end portion in the direction of insertion.

Consequently, the beam crosses a plane defined by the longitudinal axis in front of the probe, thereby avoiding any blind spots in front of the probe and facilitating the measurement on the entire surface of the cavity in a single mode of operation. Furthermore, the crossing of the emitted rays cause a centre spot to show up on the detector which may be displayed on the screen, thereby providing a visual control when the probe gets close to a surface, for example the tympanic membrane.

Since the radiation is directed to the cavity at an acute angle, i e an angle larger than 0° and smaller than 90°, the distance measurement is performed at a location of the cavity wall that lies at an angle in front of the probe, thereby allowing measurements to be taken even at positions close to the end wall of the cavity or of a constriction/narrowing of the cavity. In particular, the apparatus provides measurements of distances both of a circumferential surface relative to the direction of insertion as well as distances to the end wall in a single mode of operation. When measuring narrow cavities such as the ear canal where movement of the probe is restricted to a longitudinal movement along a direction of insertion, this may be of particular advantage.

It is a further advantage that the measurement is not impaired by additional light beams used for position monitoring of the probe.

Embodiments of the apparatus described herein use electromagnetic radiation such as light to determine the distance from the tip of the probe to the internal wall of the cavity. Based on a determined position of the probe, this information may be used to generate information about the shape of the cavity In some embodiments, e.g. when the cavity walls are human skin, e.g. in the case of a human ear canal, it may be advantageous to use light in the wavelength range of approximately 400 - 700 nm, even though electromagnetic radiation in other wavelength ranges may be suitable, e.g. visible light, infra-red light, ultra-violet light, or other parts of the electromagnetic spectrum. Different wavelength ranges may be suitable depending on the material of the cavity walls. The wavelength range between 400 - 600 nm is especially suitable for measuring the ear canal, because of the optical properties of skin, e.g. the light penetration is lower in this range.

During use of the apparatus, the inside of the cavity need not be touched during measurement, and this is advantageous for at least two reasons. Firstly, the internal surface of the cavity may be very sensitive, as is e.g. the case for the ear canal such that touching thereof may be unpleasant for the patient. Secondly, the cavity may deform when touched, and this might disturb the measured distance values and thereby corrupt the obtained data model.

The acute angle may be selected to be larger than 0° and smaller than 90°. Larger angles, as measured between the light beam and the direction of insertion - for example angles lager than 20°, such as larger than 30°, e.g. larger than 40° - generally provide a higher resolution of the distance measurement. Smaller angles on the other hand, for example angles smaller than 80° such as smaller than 70° e.g. smaller than 60°, avoid a limitation of the area in front of the probe in which measurements can be performed Such smaller angles prevent the emitted radiation from being obstructed by protruding parts of the tip of the probe, e.g. by protruding features of a light-emitting lens. The choice of a particular angle may further depend on the size of the probe and the expected typical dimensions of the cavities to be measured.

When the radiation directing means is adapted to direct a plurality of beams of radiation from the distal end portion to respective locations on the internat surface, the distance to a plurality of points on the internal surface can be measured simultaneously, thus reducing the time required for a complete mapping of the surface.

The direction of insertion may be defined by the shape of the probe and/or the position of the radiation directing means at the insertable end portion. In some embodiments, the direction of insertion is substantially parallel to the optical axis of the radiation direction means and/or the radiation receiving means. In some embodiments, the probe defines a longitudinal axis projecting out of the end portion along the direction of insertion. When the probe has an elongated shape, e.g. a rod, it is particularly suitable for insertion into a narrow cavity such as an ear canal or other tubular or canal-like cavity.
It is an advantage of the apparatus and method described herein that it allows obtaining geometrical data of surfaces of narrow passages even when such a narrow passage has a diameter smaller than the diameter of the probe.

The radiation detecting means may comprise an array of light sensitive elements such as CCD elements, thereby providing a location-sensitive detection of the reflected light

In one embodiment, the apparatus includes a radiation receiving element, e g. a lens or lens system, for receiving the reflected radiation and directing it on the radiation detecting means. In particular, when the radiation receiving element defects the received radiation to different positions of the radiation detecting element responsive to the incident angle, the incident angle of the received radiation can be easily determined from corresponding position coordinates of the detected radiation by the radiation detecting means.

When the distal end portion includes a lens having a peripheral portion for directing the radiation to the at least one location, and a central portion for receiving the reflected radiation, a particularly compact probe is provided having an end portion that combines emission and reception of radiation in an efficient way.

When the central portion incudes a radiation receiving surface, and the peripheral portion includes an annular radiation emitting surface, the annular radiation emitting surface being inclined relative to the radiation receiving portion, a large radiation emitting surface is provided for emitting radiation in a cone, thereby allowing simultaneous measurements at different positions along a circumference of the inner surface.

When the peripheral portion further includes an annular radiation reflecting surface for directing a longitudinal beam of radiation towards the radiation emitting surface, a particularly compact lens is provided that guides radiation received from a radiation source, e.g from one or more light guides emitting light in a longitudinal direction towards the lens, e.g towards an annular rear surface of the peripheral portion

In one embodiment, position data about a position of the probe with respect to a reference system are obtained using transducing means transmitting a magnetic field associated with the distal portion of the probe and second transducing means fixed relative to the head of the patient and detecting the magnetic field generated by the transmitter. The use of this method of obtaining the position data is very precise. Further it is possible to make the measurement noise insensitive. Also the transmitter of the magnetic field may be made small, so that it may easily be build into the tip of the probe.

In one embodiment the probe has a flexible part and is capable of bending. This has the advantage that the probe is capable of assuming the shape of the cavity, e.g. the ear canal. For example, this makes it possible to insert and retract the probe the full length of the ear canal as the probe continually assumes the shape of the ear canal. The ear canal of especially elderly people may have sharp bends, and by using the apparatus described herein, the probe may be carefully maneuvered past such bends as data are recorded, and without making impressions in the tissue of the ear canal, which might corrupt the measurements

When the apparatus further comprises at least one radiation guide for transmitting radiation from a proximal end of the elongated probe to the distal end portion, the radiation source may be positioned outside the cavity, thereby keeping the insertable part of the probe small. When the apparatus comprises a plurality of such radiation guides, each for transmitting radiation to a respective exit position on the distal end portion, a simultaneous illumination of different sections of the cavity wall is provided

When the apparatus comprises a radiation receiving member for receiving the reflected radiation at the distal end portion, and a radiation guide for transmitting the received radiation to the detection means, the detection means may be placed outside the cavity, so as to reduce the dimensions of the insertable part of the probe.

When the means for detecting includes means for detecting an incident angle of the reflected radiation, e.g. relative to the direction of insertion and/or the optical axis of the radiation receiving means, and when the means for determining is adapted to determine the distance from the incident angle, an efficient and accurate distance measurement is provided, e.g. by triangulation, that determines distance components at least in a transversel/radial direction relative to the direction of insertion. Hence, geometrical data relating to the circumferential surface of a cavity relative to the direction of insertion can accurately and efficiently be determined.

Embodiments of the present invention can be implemented in different ways, including the apparatus described above and in the following, a method, and further product means, each yielding one or more of the benefits and advantages described in connection with the first-mentioned apparatus, and each having, one or more embodiments corresponding to the embodiments described in connection with the first-mentioned apparatus and/or disclosed in the dependent claims.

In particular, according to one aspect, a method is disclosed herein for obtaining geometrical data relating to the internal surface of a cavity facilitates the generation of an exact model of the interval surface of the cavity.

### BRIEF DESCRIPTION OF THE DRAWINGS

Fig. 1 shows a schematic view of an example of an apparatus for obtaining geometrical data relating to the internal surface of a cavity.
Fig. 2 shows a schematic view of another example of an apparatus for obtaining geometrical data reflating to the internal surface of a cavity.
Fig. 3 shows a sectional view of the distal end of a probe of an apparatus for obtaining geometrical data relating to the internal surface of a cavity.
Fig 4 shows a sectional view of an example of a tens system of an apparatus for obtaining geometrical data relating to the internal surface of a cavity.
Fig. 5 is a side view showing the human ear and illustrating the use of the apparatus described herein.
Fig. 6 shows a schematic view of another example of an apparatus for obtaining geometrical data relating to the internal surface of a cavity

Throughout the drawings, equal reference signs refer to equal or corresponding elements, features, or components

### DETAILED DESCRIPTION

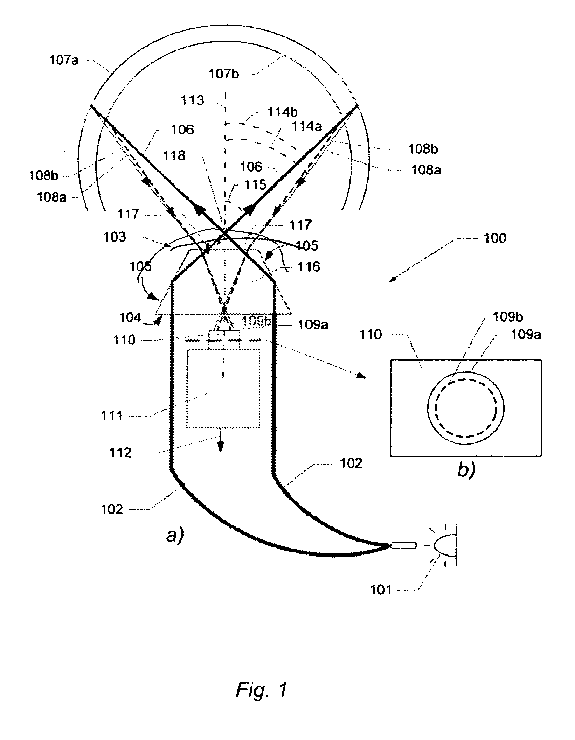

Fig. 1 a shows a schematic view of an example of an apparatus for obtaining geometrical data relating to the internal surface of a cavity. The apparatus, generally designated 100, comprises a distal end portion 103 including an optical system 116 for emitting and receiving light, a light source 101 and light guides 102 for directing the light from the light source 101 to a rear surface 104 of the optical system 116. The optical system 116 may comprise one or more lenses and/or one or more reflecting surfaces 105 for directing the light from the light guides 102 as one or more beams 106 to the internal surface 107a, 107b of a cavity.

The light beams 106 are emitted at an acute angle 115 relative to the optical axis 113 of the optical system 116 which also defines the direction of insertion of the distal end portion 103 into the cavity. The light beams 106 are emitted from respective exit positions 117 radially displaced from the optical axis. Furthermore, the exit position 117 and the position where the light beam intersects with the cavity wall 107a, b, are positioned on opposite sides of the optical axis 113. The light beams 106 thus cross the optical axis. Furthermore, the light beams 106 intersect at a point 118 in front of the probe; in the example of fig. 1 the beams intersect with each other on the optical axis.

In the example of fig. 1, two light guides 102 are shown However, it will be appreciated that generally a different number of light guides may be used, e.g. 3, 4, 5, 6, or even a larger number of light guides In some embodiment a uniform illumination over a full circumference is achieved by providing a relatively large number, e.g. between 60 or 80, optical fibres. The fibres are divided into bundles, e.g. from 4 to 6 bundles, and each bundle is illuminated with a respective light source.

For example, the light guides may be arranged such that the emitted light beams 106 intersect with each other as shown in figure 1, so as to define a double cone where the point of intersection 118 defines the apex of the cone. In the example of fig. 1, the beams 106 intersect each other on the optical axis.

The emitted light beams 106 are reflected from the internal surface 107a, b of the cavity and at least a portion of the reflected light will be reflected back in the direction of the distal end portion 103 as indicated by reflected beams 108a, b in fig. 1. The optical system 116 of the distal end portion 103 thus also functions as a light receiving system and receives the reflected light 108a, b from the cavity walls 107a, b. The optical system 116 directs the received light 108a, b towards a light sensitive element 110, e.g. an array of light sensitive elements such as a CCD, CMOS or another position sensitive light detector, referred to as a CCD in the following.

Generally a black and white CCD is more sensitive than a colour sensitive CCD element, and it is preferred when the signal-to-noise ratio becomes critical. If a monochromatic light source is used the advantage of using a colour sensitive CCD element is limited and a black and white is preferred. If a white light source is used it could be interesting to detect the colour information with a colour sensitive CCD element, as it may give a better identification of the surface of the ear canal and structures like earwax or the tympanic membrane than a black and white CCD. However both detector types may be able to indirectly detect foreign objects such as earwax, because earwax will result in a decreased reflection of the light compared to the naked skin of the ear canal.

Furthermore, the detected signal from the CCD may be displayed on a display, via an eyepiece, or the like, as a received image, thus allowing the probe described herein to be used in a fashion similar to an endoscope Such an image may be valuable for the person conducting an ear scan, e.g. for a visual inspection of the measured cavity and/or so as to provide a visual control as to how close the probe is to the end wall, e.g. the tympanic membrane. The endoscope like feature is possible when the optical system is designed as an imaging tens system with a suitable field of depth, which may be improved by an aperture in the lens system. The aperture also reduces stray light and thereby increasing the signal-to-noise ratio.

The light source 101 may be any suitable light sources, e.g. one or more incandescent lamps, light emitting diodes (LED) or diode lasers. The choice of light source is dependent on the properties of the measured object, the power needed, and noise considerations. Coupling of the light source into the light guides makes the diode lasers the most obvious choice because of a high coupling power, but modal noise or speckle may occur and only monochromatic sources are available. LEDs or incandescent lamps requires a higher output power for coupling, but modal noise is not present and both short and broad band sources e g white light can be chosen..

In fig. 1, the cavity surface 107a, b is shown as an example in a first distance at 107a from the tip of the probe and in a second distance at 107b closer to the tip of the probe. Light reflected from the surface at 107a will enter the optical system 116 as light 108a at an incident angle 114a relative to the optical axis, while light reflected from the surface at 107b will enter the optical system 116 as light 108b at an incident angle 114b relative to the optical axis. Consequently, the optical system 116 directs the incoming light 108a and 108b to respective positions 109a and 109b on the light sensitive element 110, thereby allowing the determination of the distance to the points 107a and 1007b in the direction of the emitted light 106 from the position of the detected light on the light sensitive element 110. Fig. 1b illustrates positions of constant distance on the light sensitive area of the detector 110 for two different distances 109a and 109b respectively.

The apparatus 100 further comprises a signal analysis circuit 111 wich generates an output signal 112, e.g an analogue signal or a digital data signal. In some embodiments, the output signal is indicative of an intensity distribution of the detected light across the light sensitive area 110 of the detector. Alternatively or additionally, the signal analysis circuit may perform additional signal processing steps, e.g. including the actual distance calculation based on an incident angle determined from the detected locations 109a and 109b. The distance may be calculated by means of a conventional triangulation resulting in a distance from the probe to the locations where the beams 106 intersect with the cavity wall 107a,b, i.e. a distance in a direction having a component in a radial direction from the optical axis 113. Alternatively, the distance calculation and/or further signal processing may be performed by a separate siginal/data processing unit, e.g. on a computer such as a PC to which the apparatus 100 may be connected.

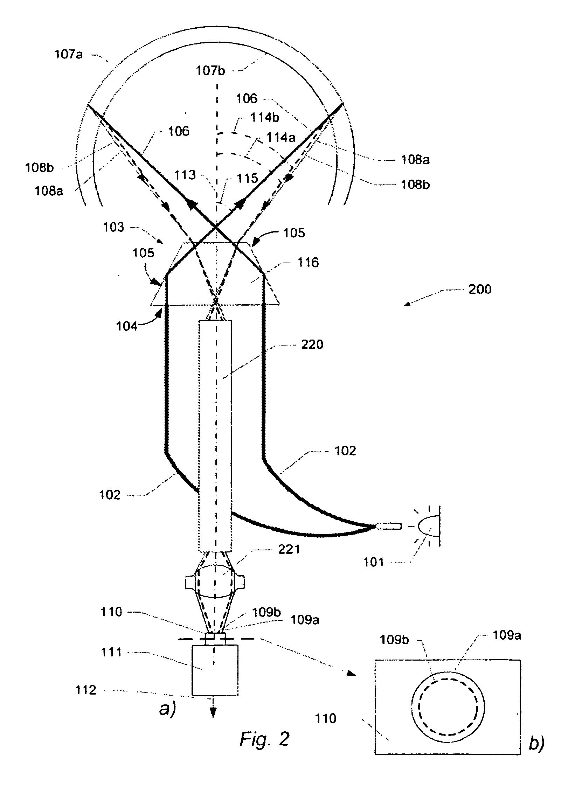

Fig. 2 shows a schematic view of another example of an apparatus, generally designated 200, for obtaining geometrical data relating to the internal surface of a cavity. The apparatus 200 is similar to the apparatus described in connection with 100, and will therefore not be described in detail again here. However, while the light sensitive element 110 of the apparatus shown in fig. 1 was arranged adjacent to the distal end portion 103, such that the reflected light is captured at the distal end of the probe, the light sensitive element 110 of the apparatus 200 is arranged remote from the distal end 103. To this end, the optical system 116 at the distal end portion 103 directs the reflected light into the distal end of a light guide 220, which in the following will be referred to as an imaging guide, e.g. an image fiber, the imaging guide 220 thus directs the received light, e g. via a further lens or lens system 221 to the light sensitive element 110.

While the apparatus of fig. 100 provides a simpler construction, the apparatus 200 does not require a light sensitive element, e.g. a CCD which is small enough to be mounted at the tip of the probe, which is going to enter the cavity. For example, the light guides 102 and the imaging guide 220 may be arranged in a flexible tube connecting the distal end portion 103 with a proximal unit including, the light source 101, the detector 110, and, optionally a signal processing unit 111.

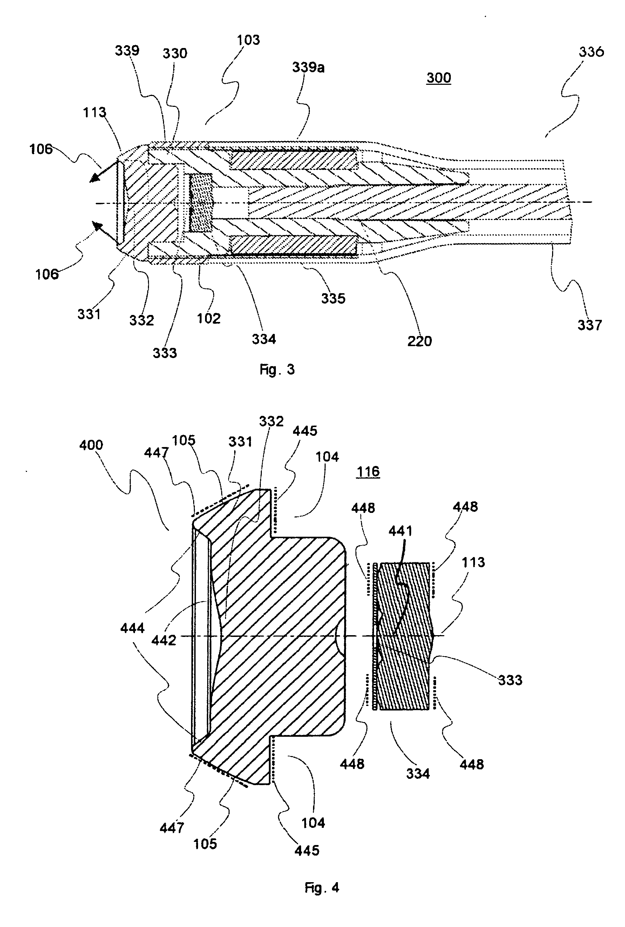

Fig 3 shows a sectional view of the distal end of a probe of an apparatus for obtaining geometrical data relating to the internal surface of a cavity. The probe, generally designated 300, has a distal light-emitting end portion 103 and a rod portion 336, which connects the distal portion to a proximal part (not explicitly shown) The rod portion 336 comprises a flexible pipe 337, a set of light guides 102 and an image guide 220. The image guide 220 is placed centrally in the pipe 337, and the light guides 102 are arranged between the pipe 337 and the image guide 220 Near the tip of the probe the image guide 220 and the light guides 102 are connected to a bushing 330 surrounded by an outer tube 339. The light guides 102 are shown at the distal portion of the probe only in fig. 3, but they extend along with the image guide 220 towards the light source 101 and the detector 110 respectively. A flexible protection cap 339a formed in unison with the pipe 337 is provided around the probe at the outer part thereof, abutting tube 339

The probe further comprises an optical system including an annular lens 331 arranged at the bushing 330 to capture the light emitted from the light guides 102 and to direct the light towards a cavity wall, e.g. as a collimated or focussed light beam 106, at an acute angle from the longitudinal axis of the probe which also defines the optical axis 113 and the direction of insertion. Light reflected from the cavity wall will enter the tube 339 through a central receiving lens 332 of the optical system. From the lens 332 the light is directed via an aperture 333 and a further imaging lens 334 towards a surface of the image guide 220 The aperture 333 increases the depth of field and prevents stray light from reaching the sensor. The light received on the surface of the image guide 220 is transmitted through the image guide 220, and will appear at the other end thereof. Here the image is captured by a CCD array (not shown). The signal from the CCD is transferred to a signal processing unit for further processing in order to calculate the distance from the probe to the canal wall. This is done by a triangulation method well known as such in the art.

In general, even though the light 106 directed towards the cavity wall may be unfocussed or uncollimated, the use of focused or collimated light provides better contrast and thus results in a more precise detection of the distance between the probe and the cavity wall

The probe 300 further comprises one or more coils 335 used to generate a magnetic field, which is picked up by sensors arranged outside the cavity so as to determine the position of the probe relative to the external sensors Thus, when the sensors are arranged in a fixed spatial relationship to the cavity, a signal/data processing unit can compute spatial coordinates of positions on the inner surface of the cavity from the optical distance measurements relative to the probe and from the position measurements of the position of the probe relative to the external sensors.

In the above embodiments, the light guides and the image guide have been shown as separate light guides. It will be appreciated that alternatively a single light guide may be used for both directing light to the tip of the probe and for transmitting the reflected light back to the CCD element. Such a combined guide may require an additional beam splitter, and may further have the disadvantage of a reduced signal to noise ratio

Hence, in the above, a probe of an apparatus has been disclosed which is suitable for obtaining geometrical data of the inner surface of a cavity, such as an ear canal.

Fig. 4 shows a sectional view of an example of a lens system of an apparatus for obtaining geometrical data relating to the internal surface of a cavity. The lens system 116 includes a front lens 400 and a rear lens 334

The front lens 400 includes an annular peripheral lens portion 331 for directing light through an annular exit surface 444 to the cavity wall surrounding the probe. The lens has an annular surface 104 on the rear side of the lens for receiving light from a light guide as described above. The light is reflected at an annular outer surface 105 towards the exit surface 444. To this end, the outer surface 105 is coated with a reflective coating as illustrated by dashed line 447. The exit surface 444 is inclined such that the emitted light is emitted at an acute angle relative to the optical axis 113. For example, in the example of fig. 4, the light is emitted from the surfaces 444 at an angle of approximately 53° relative to the optical axis 113. However, other acute angles may be selected as well, in particular angles small enough to avoid the emitted light to be obstructed by the inclined exit surface 444 on the opposite side of the lens.

The front lens 400 further includes a central lens portion 332 for receiving reflected light through a front surface 442 from the cavity wall surrounding the probe. The central lens portion 332 directs the received light toward a central portion 441 of the rear lens 334.

The exposed spaces of the front lens 400, i.e the outer surface 105 and the front surface 442 are optionally coated with a protective coating (not shown). The front lens 400 may be mounted by gluing its annual rear surface to a bushing of the probe, e.g. bushing 330 shown in fig 3. The annular rear surface for mounting is illustrated by dashed lines 445 in fig. 4.

The lends may be manufactured from any suitable material like glass or plastics. Suitable plastics materials could be polymethylmethacrylate (PMMA), cyclic olefin copolymer (COC) like Topas, cyclic orefin polymer COP like Zeonex, or the like. The reflective coating may be any same reflective material, e.g. aluminium. The protective coating may be any suitable protective material, such as quartz (SiO₂). If some of the lens features have a low radius of curvature, the choice of plastics as lens material is preferred due to the less strict requirements of the production process. For example, the lenses may be produced by an injection molding process.

The rear lens 334 includes a central lens portion 441 for receiving the light from the central lens portion 332 of the front lens and for focussing the received light onto the end surface of a light guide, e.g. image guide 220 shown in fig. 3. The rear lens may be mounted by gluing it to a corresponding bushing via annular mounting surfaces 448.

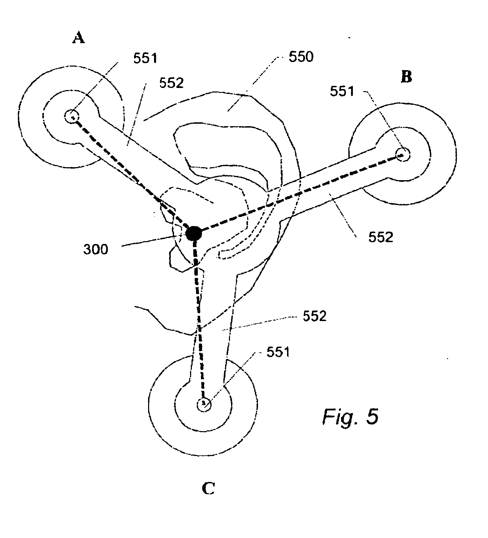

Fig. 5 is a side view showing the human ear and illustrating the use of the apparatus described herein. In use the probe 300 is gently inserted into the ear 550, and magnetic sensors 551 are placed in close relation to the patient's head. Placing the probe in the ear is done while objects in front of the probe are monitored as described above A real picture may be obtained, and/or the distance to the tympanic membrane is measured as described herein. The picture captured this way is displayed on a monitor, such that the operator may know when the probe is approaching the tympanic membrane. Once the region near the tympanic membrane is reached, the measurements may commence. This may be done while retracting the probe as corresponding values of the distances to the canal wall and the position of the probe are recorded. The recording is continued until the probe reaches the outer regions of the outer ear

In the example of fig. 5, at each sensor position A, B and C two or more sensors 551 are located, which are designed to register the magnetic field vector in each of their positions. Through this arrangement the exact location and orientation of the tip of the probe can be determined at any time. In the case shown in fig. 5, the probe 300 is located inside the canal of a human ear 550, shown schematically in the figure. The three sensor locations are arranged in a fixed construction, which in use is held immobilized relative to the parent's head In the embodiment shown in fig. 5, the fixed construction comprises a tripod, whereby each of the sensor positions are placed at the outer end of each of the branches 552 of the tripod. In use, a coil at the tip of the probe is divert at a fixed frequency and by using a lock-in procedure, thereby allowing any noise coming from other magnetic fields in the surroundings to be cancelled out from the sensor signals.

As shown in figs 1b and 2b, the image recorded by the CCD resembles a closed line having a generally circular shape with centre in the middle of the image. However, depending on the position of the probe in the ear, the circle can be more or less complete. Furthermore, the shape can also be elliptical.

The distances of points on the closed line may be automatically detected by determining intensity profiles along radial lines from the centre of the CCD, i e. the point where the optical axis intersects the image area. A set of profiles are sampled from the centre of the image and out, with the angle ranging from 0° to 360°. For each profile, the point with maximum image intensity is located, e.g by fitting a cubic-spline or other functional shape to the profile and analytically determining the top-point of the spline, thus allowing the location of the radius with sub-pixel accuracy.

Foreign objects such as hair or earwax in the ear canal may corrupt the obtained data and thus the data model of the ear canal. This may be avoided by analysing the reflected radiation in order to recognize such objects, e.g. by means of a spectral composition of the light received at detecting means. Such objects may thus be left out of the data model, thereby improving the accuracy of the model. Furthermore, the use of light makes it possible to obtain very precise data

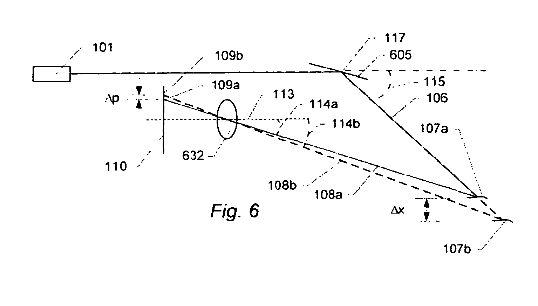

Fig. 6 shows a schematic view of another example of an apparatus for obtaining geometrical data relating to the internal surface of a cavity.
The apparatus includes a light source 101, a lens 632, and a mirror or other reflecting member 605 for directing light 106 from the light source at an acute angle 115 relative to the optical axis 113 towards the surface 107a, b of a cavity. The reflective member 605 directs the light beam 106 from an exit positions 117 at a radial distance from the optical axis 113 such that the light beam 106 crosses the optical axis 113, or at least a plane through the optical axis, before reaching the cavity wall 107a, b. The lens 632 is configured to direct light 108a, b reflected from the surface 107a, b to respective locations 109a, b on a detector 110. In the example of fig 6, the cavity wall 107a, b is shown at two radial distances from the optical axis, where the difference in radial distances is denoted Δx. The light reflected from the cavity wall at these distances is incident on the lens 632 at respective angles 114a and 114b The lens images the corresponding reflected light 108a and 108b on respective positions 109a, b at different distances from the optical axis 113. The distance between the image points 109a and 109b is denoted Δp Hence, the distances Δp is a measure of the distance Δx, as the coordinates in the image plane 110 of the reflected light from the object is given by the angle 114a,b of incidence on the lens 632 in front of the detector, e g a CCD chip.

Although some embodiments have been described and shown in detail the invention is not restricted to them, but may also be embodied in other ways within the scope of the subject matter defined in the following claims.

For example, the apparatus and method described herein have mainly been described with reference to the obtaining of a date model of the ear canal However, it will be appreciated that the apparatus and method described herein may also be applicable in connection with other cavities, such as other body cavities of the human or animal body, e g. dental cavities, nasal cavities, the urethra, etc. Furthermore, the method may be used to obtain other types of geometrical data based from the distance measurements obtained by the apparatus and method described herein. For example, the method and apparatus may be used to determine measurements of the size/diameter along an elongate cavity, e g. for detecting narrowing or other irregularities, for quality control, for medical or other purposes.

Embodiments of the method described herein can be implemented by means of hardware comprising several distinct elements, and/or at teast in part by means of a suitably programmed microprocessor. In the apparatus claims enumerating several means, several of these means can be embodied by one and the same element, component or item of hardware. The mere fact that certain measures are recited in mutually different dependent claims or described in different embodiments does not indicate that a combination of these measures cannot be used to advantage

It should be emphasized that the term "comprises/comprising" when used in this specification is taken to specify the presence of stated features, integers, steps or components but does not preclude the presence or addition of one or more other features, integers, steps, components or groups thereof.

## Claims

1. An apparatus for obtaining geometrical data relating to an internal surface of an ear canal, the apparatus comprising:
- a probe (300) having an end portion (103) insertable into the ear canal in a direction of insertion;
- wherein the probe defines a longitudinal axis projecting out of the end portion along the direction of insertion;
- radiation directing means (116, 605, 105, 331) adapted for directing light from a source through the end portion to at least one location on the internal surface (107a, 107b) of the ear canal to cause the light to be reflected from said location;
- detection means (110) for detecting the light (108a, 1 08b) from the at least one location;
- means (111) for determining from the detected light a distance between the probe and the internal surface in a direction having at least a transverse component relative to the direction of insertion;
- the radiation directing means is adapted to direct the light at an acute angle (115) relative to the direction of insertion;
wherein the radiation directing means is adapted to direct a plurality of light beams from respective exit positions radially displaced from the longitudinal axis and located at the distal end portion to respective locations on the internal surface,
and the plurality of light beams interacting in a position (118) displaced from the distal end portion in the direction of insertion so as to define a cone around the direction of insertion.

2. An apparatus according to claim 1, wherein the plurality of light beams define a double cone having an apex (118) displaced from the distal end portion in the direction of insertion.

3. An apparatus according to any one of claims 1 and 2 , wherein the acute angle is between 20 and 80 degrees, such as between 30 and 70 degrees, for example between 40 and 60 degrees.

4. An apparatus according to any one of claims 1 through 3, wherein the distal end portion includes a lens (400) having a peripheral portion (331) for directing the light radiation to the at least one location and a central portion (332) for receiving the reflected light radiation.

5. An apparatus according to claim 4, wherein the central portion includes a light radiation receiving surface (442), and wherein the peripheral portion includes an annular light radiation emitting surface (444), the annular light radiation emitting surface being inclined relative to the light radiation receiving portion.

6. An apparatus according to claim 5, wherein the peripheral portion further includes an annular reflecting surface (105) for directing a longitudinal light beam light towards the radiation emitting surface.

7. An apparatus according to any one of claims 1 through 6, further comprising at least one light guide (102) for transmitting light from a proximal end of the elongated probe to the distal end portion.

8. An apparatus according to claim 7, comprising a plurality of light guides, each for transmitting radiation to a respective exit position on the distal end portion.

9. An apparatus according to any one of claims 1 through 8, wherein the detection means includes means (110) for detecting an incident angle (1141, 114b) of the reflected light, and wherein the means for determining (111) is adapted to determine the distance from the incident angle.

10. An apparatus according to any one of claims 1 through 9, wherein the detection means (110) includes an array of light sensitive detector elements.

11. An apparatus according to any one of claims 1 through 10, wherein the light has a wavelength between 400 and 700nm, preferably in the range between 400 and 600nm.

12. An apparatus according to any one of claims 1 through 11, wherein the ear canal is of a human or animal body.

13. A Method for obtaining geometrical data relating to an internal surface of an ear canal using the apparatus of claims 1-12.

14. A method as claimed in claim 13, further comprising obtaining position data indicative of a position of the probe inside the ear canal.

## Patentansprüche

1. Vorrichtung zum Erhalten geometrischer Daten in Bezug auf eine innere Oberfläche eines Gehörgangs, wobei die Vorrichtung umfasst:
- eine Sonde (300) mit einem Endabschnitt (103), der in einer Einsetzrichtung in den Gehörgang einsetzbar ist;
- wobei die Sonde eine Längsachse definiert, die sich über den Endabschnitt hinaus entlang der Einsetzrichtung erstreckt;
- ein Strahlungsrichtmittel (116, 605, 105, 331), das zum Richten von Licht von einer Quelle durch den Endabschnitt zu zumindest einem Ort an der inneren Oberfläche (107a, 107b) des Gehörgangs ausgebildet ist, um zu bewirken, dass Licht von dem Ort reflektiert wird;
- ein Detektionsmittel (110) zum Detektieren des Lichts (108a, 108b) von dem zumindest einem Ort;
- ein Mittel (111) um aus dem detektierten Licht einen Abstand zwischen der Sonde und der inneren Oberfläche in einer Richtung zu bestimmen, die zumindest eine Querkomponente bezüglich der Einsetzrichtung hat;
- wobei das Strahlungsrichtmittel ausgebildet ist das Licht in einem spitzen Winkel (115) bezüglich der Einsetzrichtung auszurichten;
wobei das Strahlungsrichtmittel ausgebildet ist, eine Vielzahl von Lichtstrahlen von entsprechenden Ausgangspositionen, die radial von der Längsachse versetzt und am distalen Endabschnitt angeordnet sind, zu entsprechenden Orten auf der inneren Oberfläche zu richten,
und die Vielzahl von Lichtstrahlen sich an einer Position (118), die von dem distalen Endabschnitt in Einsetzrichtung beabstandet ist, schneiden, so dass sie einen Konus um die Einsetzrichtung definieren.

2. Vorrichtung gemäß Anspruch 1, bei dem die Vielzahl der Lichtstrahlen einen Doppelkonus definiert, der einen Scheitelpunkt (118) hat, welcher in Einsetzrichtung von dem distalen Endabschnitt beabstandet ist.

3. Vorrichtung gemäß einem der Ansprüche 1 und 2, bei der der spitze Winkel zwischen 20 und 80 Grad beträgt, wie etwa zwischen 30 und 70 Grad, z. B. zwischen 40 und 60 Grad.

4. Vorrichtung gemäß einem der Ansprüche 1 bis 3, bei dem der distale Endabschnitt eine Linse (400) mit einem peripheren Abschnitt (331) zum Richten der Lichtstrahlung auf den zumindest einen Ort und einen zentralen Abschnitt (332) zum Empfangen der reflektierten Lichtstrahlung einschließt.

5. Vorrichtung gemäß Anspruch 4, bei dem der zentrale Abschnitt eine lichtstrahlungsempfangende Oberfläche (442) einschließt und bei der der periphere Abschnitt eine ringförmige lichtstrahlungsaussendende Oberfläche (444) einschließt, wobei die ringförmige lichtstrahlungsaussendende Oberfläche bezüglich der lichtstrahlungsempfangenden Oberfläche geneigt ist.

6. Vorrichtung gemäß Anspruch 5, bei der der periphere Abschnitt außerdem eine ringförmige lichtreflektierende Oberfläche (105) zum Richten eines Längslichtstrahls auf die strahlungsaussendende Oberfläche einschließt.

7. Vorrichtung gemäß einem der Ansprüche 1 bis 6, weiterhin aufweisend zumindest einen Lichtleiter (102) zum Übertragen von Licht von einem proximalen Ende der länglichen Sonde zu dem distalen Endabschnitt.

8. Vorrichtung gemäß Anspruch 7, mit einer Vielzahl von Lichtleitern, jeder zum Übertragen von Strahlung zu einem entsprechenden Austrittssort auf dem distalen Endabschnitt.

9. Vorrichtung gemäß einem der Ansprüche 1 bis 8, bei der das Detektionsmittel ein Mittel (110) zum Detektieren eines Einfallwinkels (1141, 114b) des reflektierten Lichts einschließt und wobei das Mittel zum Bestimmen (111) ausgebildet ist, den Abstand aus dem Einfallswinkel zu bestimmen.

10. Vorrichtung gemäß einem der Ansprüche 1 bis 9, bei dem das Detektionsmittel (110) eine Matrix von lichtempfindlichen Detektorelementen einschließt.

11. Vorrichtung gemäß einem der Ansprüche 1 bis 10, bei dem das Licht eine Wellenlänge zwischen 400 und 700 nm hat, vorzugsweise im Bereich zwischen 400 und 600 nm.

12. Vorrichtung gemäß einem der Ansprüche 1 bis 11, bei dem der Gehörgang ein Gehörgang eines menschlichen oder tierischen Körpers ist.

13. Verfahren zum Erhalten geometrischer Daten in Bezug auf eine innere Oberfläche eines Gehörgangs unter Verwendung der Vorrichtung gemäß der Ansprüche 1 bis 12.

14. Verfahren gemäß Anspruch 13, weiterhin aufweisend Erhalten von Positionsdaten hinsichtlich einer Position der Sonde innerhalb des Gehörgangs.

## Revendications

1. Appareil pour obtenir des données géométriques relatives à une surface interne d'un conduit auditif, l'appareil comprenant :
- une sonde (300) comportant une partie d'extrémité (103) pouvant être inséré dans le canal de l'oreille dans une direction d'insertion;
- dans lequel la sonde définit un axe longitudinal faisant saillie de la partie d'extrémité le long de la direction d'insertion;
- un moyen de direction de rayonnement (116, 605, 105, 331) adapté pour diriger la lumière provenant d'une source à travers la partie d'extrémité à au moins un emplacement sur la surface interne (107a, 107b) du conduit auditif pour amener la lumière à être réfléchie par ledit emplacement;
- un moyen de détection (110) pour détecter la lumière (108a, 108b) à partir dudit au moins un emplacement;
- un moyen (111) pour déterminer à partir de la lumière détectée une distance entre la sonde et la surface interne dans une direction ayant au moins une composante transversale par rapport à la direction d'insertion;
- le moyen de direction de rayonnement est adaptée pour diriger la lumière selon un angle aigu (115) par rapport à la direction d'insertion;
dans lequel le moyen de direction de rayonnement est adapté pour diriger une pluralité de faisceaux de lumière à partir de positions de sortie respectives décalées radialement de l'axe longitudinal et situées au niveau de la partie d'extrémité distale à des emplacements respectifs sur la surface interne,
et la pluralité de faisceaux de lumière se croisant en une position (118) déplacée à partir de la partie d'extrémité distale dans la direction d'insertion de manière à définir un cône autour de la direction d'insertion.

2. Appareil selon la revendication 1, dans lequel la pluralité de faisceaux de lumière définit un double cône ayant un sommet (118) déplacé à partir de la partie d'extrémité distale dans la direction d'insertion.

3. Appareil selon l'une quelconque des revendications 1 et 2, dans lequel l'angle aigu est compris entre 20 et 80 degrés, tel que entre 30 et 70 degrés, par exemple entre 40 et 60 degrés.

4. Appareil selon l'une quelconque des revendications 1 à 3, dans lequel la partie d'extrémité distale comprend une lentille (400) ayant une partie périphérique (331) pour diriger le rayonnement de lumière audit au moins un emplacement et une partie centrale (332) pour recevoir le rayonnement lumineux réfléchi.

5. Appareil selon la revendication 4, dans lequel la partie centrale comporte une surface de réception de rayonnement de lumière (442), et dans lequel la partie périphérique comprend une surface annulaire d'émission de rayonnement de lumière (444), la surface annulaire d'émission de rayonnement de lumière étant inclinée par rapport à la partie de réception de rayonnement de lumière.

6. Appareil selon la revendication 5, dans lequel la partie périphérique comprend en outre une surface annulaire réfléchissante de lumière (105) pour diriger un faisceau lumineux longitudinal vers la surface d'émission de rayonnement.

7. Appareil selon l'une quelconque des revendications 1 à 6, comprenant en outre au moins un guide de lumière (102) pour transmettre la lumière d'une extrémité proximale de la sonde allongée à la partie d'extrémité distale.

8. Appareil selon la revendication 7, comprenant une pluralité de guides de lumière, chacun pour transmettre un rayonnement à une position de sortie respective sur la partie d'extrémité distale.

9. Appareil selon l'une quelconque des revendications 1 à 8, dans lequel le moyen de détection comprend un moyen (110) pour détecter un angle d'incidence (1141, 114b) de la lumière réfléchie, et dans lequel le moyen de détermination (111) est adapté pour déterminer la distance à partir de l'angle d'incidence.

10. Appareil selon l'une quelconque des revendications 1 à 9, dans lequel le moyen de détection (110) comprend un réseau d'éléments détecteurs sensibles à la lumière.

11. Appareil selon l'une quelconque des revendications 1 à 10, dans lequel la lumière a une longueur d'onde entre 400 et 700 nm, de préférence dans la plage entre 400 et 600 nm.

12. Appareil selon l'une quelconque des revendications 1 à 11, dans lequel le canal auditif est celui d'un corps humain ou animal.

13. Procédé pour obtenir des données géométriques relatives à une surface interne d'un conduit auditif, en utilisant l'appareil des revendications 1-12.

14. Procédé selon la revendication 13, comprenant en outre l'obtention de données de position indicatives d'une position de la sonde à l'intérieur du conduit auditif.
